# EUROPEAN PATENT APPLICATION

(11) **EP 3 674 293 A1**
(43) Date of publication of application: **01.07.2020**
(21) Application number: 18216012.7
(22) Date of filing: 31.12.2018
(51) Int. Cl.: C07D 251/70

(54) **PROCESS FOR SYNTHESIZING S-TRIAZINE COMPOUNDS**

(71) Applicant: Clariant International Ltd, 4132 Muttenz (CH)
(72) Inventor: MEHTA, Hiten Sharadchandra, 400708 Airoli, Navi Mumbai (IN); RAGHUVEER, Tallam, 400708 Airoli, Navi Mumbai (IN); RAMESH, Karmakonda, 400708 Airoli, Navi Mumbai (IN); SRINIVAS, Uppalanchi, 400708 Airoli, Navi Mumbai (IN); PAVANKUMAR, Duddilla, 400708 Airoli, Navi Mumbai (IN)
(74) Representative: Kampen, Daniela

(57) **Abstract**

According to the invention, a process for synthesizing a UV filter having the Formula X is provided: 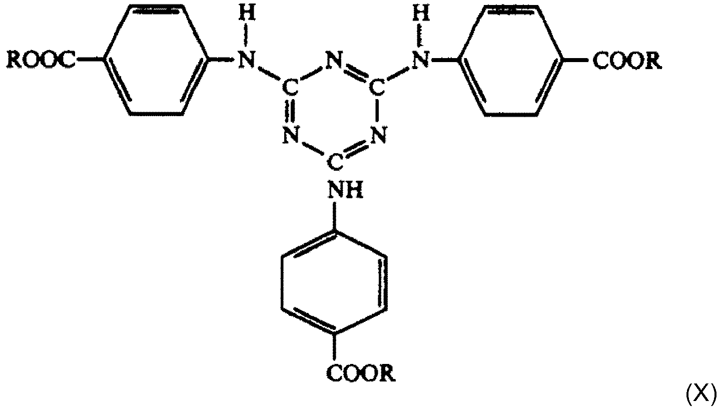 where R is independently selected from linear or branched aliphatic groups having 1 to 20 carbon atoms, the process comprising:
(i) Esterifying p-nitrobenzoic acid by reacting it with a linear or branched-chain alcohol having from 1 to 20, preferably from 4 to 10, carbon atoms;
(ii) Reducing the p-nitrobenzoic acid ester from (i) in an aprotic organic solvent to produce a p-aminobenzoic acid ester;
(iii) Reacting the p-aminobenzoic acid ester from (ii) with a less than stoichiometric amount of cyanuric halide to produce a compound according to Formula X.

## Description

The present invention relates to a process for synthesizing s-triazine compounds. These materials may be used as sunscreens.

A process for the manufacture of the s-triazine compound, octyl triazone, is known from Chinese patent application CN104860893 A. The starting materials are p-nitrobenzoic acid and 2-ethylhexanol and the process includes the steps of:
- Esterification of the starting materials to synthesize ethylhexyl p-nitrobenzoate;
- Reduction of the ethylhexyl p-nitrobenzoate using ammonium chloride/powdered iron catalyst in a methanol/water, ethanol/water or acetone/water solvent to produce ethylhexyl p-aminobenzoate;
- Islolation of the ethylhexyl p-aminobenzoate via filtration and concentration under vacuum;
- Reaction of the ethylhexyl p-aminobenzoate with cyanuric chloride in acetone solvent to synthesize an intermediate material;
- Isolation of the intermediate material via vacuum filtration and drying;
- Reaction of ethylhexyl p-aminobenzoate with the intermediate material to synthesize octyl triazone.

This process is complex and time-consuming, because several intermediate isolation stages are required and because some of the individual stages themselves are very long. The reduction step alone lasts for 16 to 17 hours. In addition, the use of different solvents in each stage requires several purifications. It would be advantageous to reduce the number of purification steps required and to use fewer solvents. It is with this background that the present invention has been devised.

According to the invention, a process for synthesizing a UV filter having the Formula X is provided: 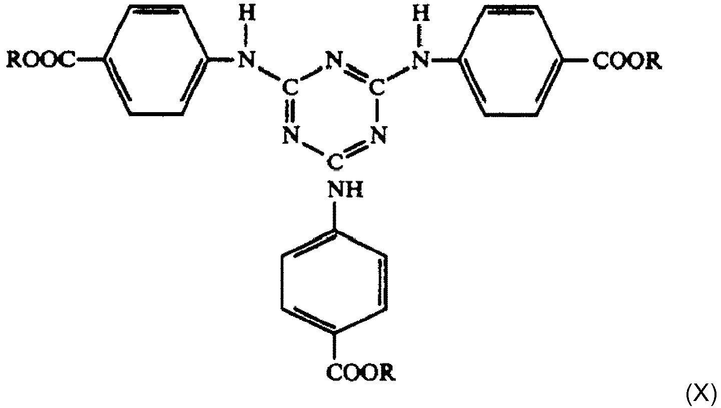 where R is independently selected from linear or branched aliphatic groups having 1 to 20 carbon atoms, the process comprising:
(i) Esterifying p-nitrobenzoic acid by reacting it with a linear or branched-chain alcohol having from 1 to 20, preferably from 4 to 10, carbon atoms;
(ii) Reducing the p-nitrobenzoic acid ester from (i) in an aprotic organic solvent to produce a p-aminobenzoic acid ester;
(iii) Reacting the p-aminobenzoic acid ester from (ii) with a less than stoichiometric amount of cyanuric halide to produce a compound according to Formula X.

As used herein, the term "aprotic" when used in relation to a solvent means that the solvent molecule does not comprise an O-H or an N-H bond.

In this document, including in all embodiments of all aspects of the present invention, the following definitions apply unless specifically stated otherwise.

All percentages are by weight (w/w) of the total composition. All ratios are weight ratios. "wt.%" means percentage by weight. References to 'parts' e.g. a mixture of 1 part X and 3 parts Y, is a ratio by weight. "QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams" and "kg" means "kilogram" or "kilograms". Herein, "comprising" means that other steps and other ingredients can be in addition. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. "Molecular weight" or "M.Wt." or "MW" and grammatical equivalents mean the number average molecular weight.

The reaction in (i) is advantageously performed in the presence of a catalyst. Preferably, the catalyst is selected from sulfuric acid, p-toluene sulfonic acid, polyphosphoric acid, thionyl chloride and mixtures thereof. The reaction in (i) is generally performed in an organic solvent which may advantageously be xylene or toluene and is preferably toluene. The reaction in (i) is generally performed under reflux at a temperature of 100 to 130 degrees Celsius, preferably 110-115 degrees Celsius. The reactants are refluxed for a period which is typically up to 15 hours, preferably from 10 to 12 hours.

With regard to (ii), the reduction may be performed in the presence of a reducing agent and a catalyst. Suitable reducing agent/catalyst pairings may be selected from iron/HCI, zinc/HCI, ammonium chloride with zinc or iron, hydrazine hydrate with iron (III) chloride or zinc chloride, iron-acetic acid and mixtures thereof.

Alternatively, with regard to (ii), the reduction may take the form of a catalytic hydrogenation under a hydrogen atmosphere with a catalyst, such as palladium, Raney Nickel or rhodium. The palladium or rhodium is advantageously deposited on a substrate, such as carbon.

The temperature of the reduction reaction is advantageously controlled to be within defined limits until it is complete.

The solvent in (ii) is an aprotic solvent. This is in contrast to the process of CN104860893 A, which employs protic solvents, such as water mixed with methanol or ethanol (which are also both polar protic solvents), or acetone, with methanol/water being preferred. Without wishing to be bound by theory, the present inventors consider the use of such protic solvents in (ii) to be disadvantageous, because these solvents, especially water, may give rise to unwanted side reactions in (iii). The presence of a drying step may not be sufficient to avoid this disadvantage, because alcohols are hygroscopic and may result in residual water remaining in the dried product. Cyanuric chloride has a tendency to hydrolyze in the presence of water in the process of triazine derivative production, which may generate unwanted side-products, such as 2,4-dichloro-6-hydroxy-s-triazine, 2-chloro-4,6,-dihydroxy-s-triazine and/or 2,4,6-dihydroxy-s-triazine(cyanuric acid) which must be removed in additional process steps.

The aprotic solvent in (ii) may comprise non-polar aprotic solvent, polar aprotic solvent or mixtures thereof. Advantageously, the aprotic solvent is a non-polar aprotic solvent selected from toluene, xylene, cyclohexane, one or more alkane(s) having a boiling point from 80 to 150 Celsius, and mixtures thereof.

Regarding (iii) the cyanuric halide in (iii) may be selected from cyanuric chloride, cyanuric bromide and mixtures thereof. Advantageously, the cyanuric halide is cyanuric chloride. The reaction in (iii) is advantageously performed under reflux at a temperature of 90 to 120 degrees Celsius, preferably 95-110 degrees Celsius. Reflux generally lasts for 1 to 15 hours, preferably 4 to 8 hours, more preferably 5 to 7 hours.

In (iii) the p-aminobenzoic acid ester is reacted with a less than stoichiometric amount of cyanuric halide to produce a compound according to Formula X. Advantageously, the molar ratio of cyanuric halide to p-aminobenzoic acid ester is 1:3 to 1:5.

In (iii), two reactions take place. In the first place, cyanuric halide reacts with some of the excess of p-aminobenzoic acid ester to form an intermediate. Secondly, remaining p-aminobenzoic acid ester reacts with the intermediate to form a compound according to Formula X. In CN104860893 A, these two reactions are performed separately: after the reaction between cyanuric halide and p-aminobenzoic acid ester, the intermediate is isolated by means of a vacuum filtration and a drying step prior to reaction with p-aminobenzoic acid ester to form a compound according to Formula X. Although the present inventors do not know exactly why this is done, it seems likely that this more complex process is needed to remove unwanted side-products which have arisen as a result of carrying out the reduction of p-nitrobenzoic acid ester in protic solvents.

The process of the present invention is simpler than the process of CN104860893 A, because the two steps involved in converting a mixture of cyanuric halide and p-aminobenzoic acid ester to a compound having Formula X may be performed in the same reaction vessel directly one after the other, without intervening process steps. In particular, there is no separation, or isolation of an intermediate, such as by filtration.

Where a solvent is required in the present process, it may be the same throughout, which is simpler, less energy intensive (since fewer solvent-removal steps are required) and may be better for the environment

Advantageously, the linear or branched-chain alcohol used in the process of the invention has from 1 to 20 carbon atoms is 2-ethylhexanol. In such a case, Formula X is 4-[[4,6-bis[[4-(2-ethylhexoxy-oxomethyl)phenyl]amino]-1,3,5-triazin-2-yl]amino]benzoic acid 2-ethylhexyl ester, or ethylhexyl triazone (octyl triazone).

Advantageously, according to the process of the invention, a compound according to Formula X is isolated in one or more further steps, such as recrystallisation in ethanol.

### LABORATORY EXAMPLES

Ethylhexyl Triazone was made according to the following methods, which illustrate the subject matter of the invention without restricting it thereto. Temperatures are room temperature unless otherwise stated. Routine additional purification steps have not been defined in detail.

### Stage I (Esterification)

| **RAW MATERIALS** | | | | |
|---|---|---|---|---|
| **Raw material** | **Quantity** | **Molecular Weight** | **Moles** | **Mole equivalent** |
| Para nitro benzoic acid (PBNA) | 1.0 kg | 167 | 5.988 | 1 |
| 2-ethyl hexanol | 0.824 kg | 130 | 6.33 | 1.058 |
| Sulfuric acid | 0.02 kg | 98 | 0.2 | 0.033 |
| Toluene | 3.5 litres | | | |

### Method

1. Charge a round bottomed flask with the toluene and sulphuric acid.
2. Add the PNBA with constant stirring.
3. Add 2-ethylhexyl alcohol.
4. Heat to reflux temperature of 110°C-115°C and maintain at reflux for 10-12 hours, while removing water to synthesize ethylhexyl p-nitrobenzoate.
5. Stop heating and cool to room temperature.
6. Purification to obtain 1.6kg of ethylhexyl p-nitrobenzoate in toluene with a total volume of 4.6 liters.

### Stage-II (Reduction)

The product of Stage I was subjected to a reduction by either A. catalytic hydrogenation or B. using a reducing agent. Determination of when the reduction was complete was performed using thin layer chromatography.

### A. Reduction by Catalytic Hydrogenation

The product of Stage I was distilled to remove the toluene and the ethylhexyl p-nitrobenzoate was dissolved in a mixture of ethanol and methanol to make up a total volume to 4.6 liters. The 4.6 liter solution of ethylhexyl-p-nitrobenzoate was placed in a hydrogenator and 32 g of 10%wt Pd/C catalyst (10%wt palladium on carbon) was added at room temperature under a nitrogen atmosphere. Hydrogen was added at a pressure from 2mbar to 5 mbar (2mBar is preferred) at room temperature. The reaction temperature was increased from room temperature to 50 degrees Celsius to 75 degrees Celsius (55 degrees Celsius is preferred) and the reaction continued for 2-3 hours. After completion of the reaction, the hydrogen gas was released and the reaction mass was cooled down to room temperature. The reaction mass was then filtered, the Pd/C was washed with 100 ml of methanol followed by 10 ml of water. The filtrate was collected and the solvents were distilled off to obtain the p-aminobenzoic acid ester product. The product was placed in 500ml of toluene and stirred for 10 minutes. After this, the product was distilled under vacuum to remove the residual ethanol/methanol. The toluene was also removed during the distillation and was replaced following distillation with pure toluene solvent. The p-aminobenzoic acid ester product (1.27kg of the ester in toluene) was obtained, which was ready for use in Stage III.

In place of Pd/C one may, for example, use the same amount (32g in this case) of Raney Nickel or Rh/C (for example 5%wt Rhodium on Carbon).

### B. Reduction using Reducing Agents

### Example 1

The 1.6kg of ethylhexyl p-nitrobenzoate in toluene (4.6 litres) from Stage I was placed in a round bottom flask. To this, add 80 g of activated carbon and 1.72g of FeCl₃ were added. To this reaction mass, 0.912 kg of 80% hydrazine hydrate was added at 50-55 degrees Celsius over a period of 1-3 hrs. The reaction temperature was then increased to 75-80 degrees Celsius and the reaction was continued for 1-2 hours to obtain a solution of ethylhexyl-p-amino benzoate in toluene (1.27 kg of ethylhexyl-p-amino benzoate in toluene). The toluene solution containing Ethylhexyl-p-amino benzoate was ready for Stage III.

### Example 2

The 1.6kg of ethylhexyl p-nitrobenzoate in toluene (4.6 litres) from Stage I was placed in a round bottom flask. To this, 1.58 kg of NH₄Cl was added and the mixture was heated to 50-55 degrees Celsius. At this temperature, 1.60 kg of Fe powder was added portion-wise over a 7-hour period. The reaction temperature was then increased to 70-75 degrees Celsius and the mixture was stirred for 2-3hrs. Following completion of the reaction, the temperature was reduced to 50-55 degrees Celsius and the mixture was filtered. The toluene layer was washed with 1 liter of water and the organic layer was separated. The organic layer contained 1.27 kg of Ethylhexyl-p-amino benzoate in solution in toluene. The toluene solution containing Ethylhexyl-p-amino benzoate was ready for Stage III.

### Example 3

The 1.6kg of ethylhexyl p-nitrobenzoate in toluene (4.6 litres) from Stage I was placed in a round bottom flask. To this, 1.35 kg of Fe powder was added and the reaction mass was heated to 80 to 85 degrees Celsius. A mixture of acetic acid/water (0.133 kg of acetic acid in 0.865 ml of water) was added portion-wise over a period of 5-6 hours. After the addition, stirring was continued for an additional 2-3 hours at 80 to 85 degrees Celsius. Following completion of the reaction, the temperature was reduced to 50-55 degrees Celsius and the mixture was filtered. The toluene layer was washed with 1 liter of water and the organic layer was separated. The organic layer contained 1.27 kg of Ethylhexyl-p-amino benzoate in solution in toluene. The toluene solution containing Ethylhexyl-p-amino benzoate was ready for Stage III.

### Example 4

The 1.6kg of ethylhexyl p-nitrobenzoate in toluene (4.6 litres) from Stage I was placed in a round bottom flask. 4.5kg of zinc powder was added followed by 3.2 litres of isopropyl alcohol. The reaction mass temperature was then cooled to 10-15 degrees Celsius, after which 11.25 litres of 36% HCI was added over 3-4 hours, maintaining the temperature below 40 degrees Celsius. The temperature was then increased to 50-55 degrees Celsius and stirred for 2-3 hours. Following completion of the reaction, the reaction mass was reduced to room temperature and the excess zinc was removed by filtration. The reaction mass was washed with 1 liter of toluene and separated into layers. The toluene layer was washed with 1 liter of water and the organic layer was separated. The organic layer contained 1.27 kg of Ethylhexyl-p-amino benzoate in solution in toluene. The toluene solution containing Ethylhexyl-p-amino benzoate was ready for Stage III.

### Example 5

The 1.6kg of ethylhexyl p-nitrobenzoate in toluene (4.6 litres) from Stage I was placed in a round bottom flask. 40 liters of water and 5.7 litres of ethanol were added together with 2.88kg ammonium chloride and the mixture was stirred for 20 min at room temperature. After this, 3.2kg of zinc powder was added over a period of 1 hour at room temperature and the mixture was stirred for a further 1-2 hours at room temperature. After completion of the reaction, excess zinc was filtered off at room temperature and washed with 1 liter of toluene. The filtrate was extracted with 3.0 liters of toluene and the layers were separated. The toluene layer was washed with 1 liter of water and the organic layer was separated. The organic layer contained 1.27 kg of Ethylhexyl-p-amino benzoate in solution in toluene. The toluene solution containing Ethylhexyl-p-amino benzoate was ready for Stage III.

### Stage III

0.304kg of cyanuric chloride was added to a flask containing the 1.27kg of ethylhexyl p-aminobenzoate in toluene from Stage II. The mixture was slowly heated to 80-85 degrees Celsius over a period of 1 hour. The heating was continued for another hour at 80-85 degrees Celsius. The mixture was then refluxed at about 100°C for a period of 6-7 hours to obtain octyl triazone. The octyl triazone was decolorized using carbon and purified using a HyflowTM bed to obtain 1.45-1.55 kg wet weight of octyl triazone.

## Claims

1. A process for synthesizing a UV filter having the Formula X: 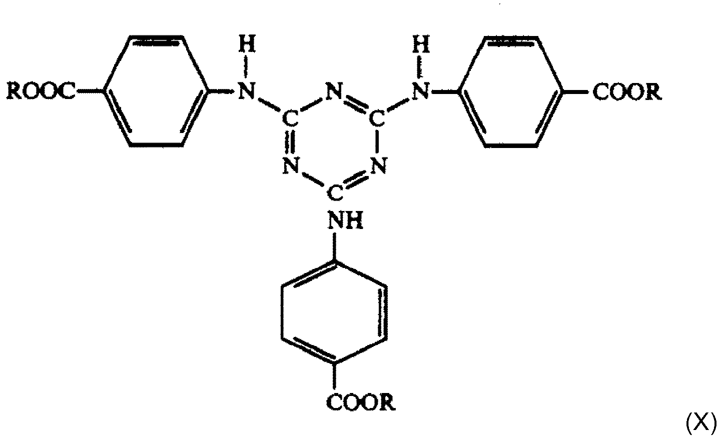 where R is independently selected from linear or branched aliphatic groups having 1 to 20 carbon atoms, the process comprising:
(i) Esterifying p-nitrobenzoic acid by reacting it with a linear or branched-chain alcohol having from 1 to 20, preferably from 4 to 10, carbon atoms;
(ii) Reducing the p-nitrobenzoic acid ester from (i) in an aprotic organic solvent to produce a p-aminobenzoic acid ester;
(iii) Reacting the p-aminobenzoic acid ester from (ii) with a less than stoichiometric amount of cyanuric halide to produce a compound according to Formula X.

2. The process of claim 1, wherein the reaction in (i) is performed in the presence of a catalyst and wherein the catalyst is preferably selected from sulfuric acid, p-toluene sulfonic acid, polyphosphoric acid, thionyl chloride and mixtures thereof.

3. The process of claim 1 or 2, wherein the reaction in (i) is performed in an organic solvent, preferably toluene.

4. The process of any preceding claim, wherein the reaction in (i) is performed under reflux at a temperature of 100 to 130 degrees Celsius, preferably 110-115 degrees Celsius.

5. The process of any preceding claim, wherein the reduction in (ii) is performed in the presence of a reducing agent and a catalyst.

6. The process of claim 5, wherein the reduction in (ii) is performed in the presence of iron/HCI, zinc/HCI, ammonium chloride with zinc or iron, hydrazine hydrate with iron (III) chloride or zinc chloride, iron-acetic acid or mixtures thereof.

7. The process of any of claims 1 to 4, wherein the reduction in (ii) comprises a catalytic hydrogenation.

8. The process of claim 7, wherein the catalyst is selected from palladium, Raney Nickel, rhodium or mixtures thereof.

9. The process of any preceding claim, wherein the aprotic solvent in (ii) comprises non-polar aprotic solvent, polar aprotic solvent or mixtures thereof.

10. The process of any preceding claim, wherein the aprotic solvent is a non-polar aprotic solvent selected from toluene, xylene, cyclohexane, one or more alkanes having a boiling point from 80 to 150 Celsius, and mixtures thereof.

11. The process of any preceding claim, wherein the cyanuric halide in (iii) is selected from cyanuric chloride, cyanuric bromide and mixtures thereof.

12. The process of any preceding claim, wherein the reaction in (iii) is performed under reflux at a temperature of 90 to 120 degrees Celsius, preferably 95-110 degrees Celsius.

13. The process of claim 12, wherein reflux lasts for 4 to 8 hours, preferably 6 to 7 hours.

14. The process of any preceding claim, wherein the molar ratio of cyanuric halide to p-aminobenzoic acid ester is 1:3 to 1:5.

15. The process of any preceding claim, wherein (iii) is performed without the separation or isolation of any intermediate.

16. The process of any preceding claim, wherein the reactants remain in the same reaction vessel throughout (iii).

17. The process of any preceding claim, wherein the linear or branched-chain alcohol having from 1 to 20 carbon atoms is 2-ethylhexanol.

18. The process of any preceding claim, additionally comprising isolating a compound according to Formula X.

19. The process of any preceding claim, wherein Formula X is ethylhexyl triazone.
